Europäisches Patentamt

(19) European Patent Office

Office européen des brevets

(11) Publication number: **0 459 788 A2**

## (12) EUROPEAN PATENT APPLICATION

(21) Application number: 91304865.8

(22) Date of filing: 29.05.91

(51) Int. Cl.⁵: **A61K 37/14, C07K 3/08,** C07K 15/00

(30) Priority: 01.06.90 GB 9012285
26.02.91 GB 9104011

(43) Date of publication of application:
04.12.91 Bulletin 91/49

(84) Designated Contracting States:
AT BE CH DE DK FR GB LI NL SE

(71) Applicant: COMMON SERVICES AGENCY
Trinity Park House, South Trinity Road
Edinburgh EH5 3SE (GB)

(72) Inventor: Pepper, Duncan Stephen, Dr.
29 Mansionhouse Road
Edinburgh EH9 2JD (GB)
Inventor: McDonald, Shirley Lynn
113 Galashiels Road
Stow, Galashiels (GB)

(74) Representative: Ede, Eric et al
Fitzpatricks 4 West Regent Street
Glasgow G2 1RS (GB)

(54) Modified proteins and methods of producing same.

(57) A stable oxygen transport medium comprising haem proteins is obtainable by treating a haem protein with a primary cross-linking reagent to achieve a significant level of protein coupling and form polymers thereof, and modifying the polymerising protein to attain an acceptable level of oxygen affinity wherein the modification is carried out during polymerisation or as a sequential step.

EP 0 459 788 A2

This invention relates to modified proteins and methods of producing same. In particular it relates to the treatment of haem proteins e.g. haemoglobins to cross-link or polymerise and stabilise them, and to modify their affinity for binding gases especially oxygen.

Considerable research has been devoted to the development of modified stroma-free haemoglobin (SFHb) solutions as potential oxygen-carrying plasma expanders. The near-normal oxygen affinity, long shelf life and potentially reduced risk of disease transmission would make such solutions uniquely suitable in situations where blood transfusion is not practical. More conventional resuscitative fluids such as albumin, hydroxyethyl starch (HES) and Ringers' lactate lack significant oxygen-carrying capacity.

However, haemoglobin (Hb) solutions have been found to cause vasoconstriction in coronary, cerebral and renal vascular beds. There are also early reports that Hb solutions, of varying degrees of purity, could produce a vasoactive response in animals. These side effects obviously severely limit or at least inhibit the clinical use of such materials.

Haemoglobin, if used in an unmodified form as an artificial oxygen carrier is rapidly excreted through the kidneys, thus meaning that continuous infusions would be necessary to have a clinical effect.

It is known that the oxygen affinity of haemoglobin can be altered and in particular decreased by various methods, including treating the Hb with pyridoxal derivatives. In addition it is also known that cross-linking haemoglobin to form dimers, trimers, tetramers etc of haemoglobin can have two effects. Firstly this can decrease its rate of renal excretion, thus increasing its half-life *in vivo* and hence its clinical utility. Secondly it is observed that the cross-linked haemoglobin tends to release its oxygen more readily than non-cross-linked haemoglobin. This latter feature can obviously be of benefit in the treatment of patients where it is desired to get oxygen to the body tissues as easily and efficiently as possible.

Those in this art will recognise that a haemoglobin "molecule" is itself a tetramer of four protein sub-units ($\alpha$ and $\beta$ sub-units) bound to each other. In this patent application "haemoglobin", unless specified otherwise, will be taken to mean this combination of $2\alpha$ and $2\beta$ sub-units and it will be understood that the literature may refer to these combined units of haemoglobin as a tetramer. However herein all references to dimers, tetramers etc, unless specifically stated to the contrary, will mean intermolecular linked multiples of the basic haemoglobin $\alpha_2\beta_2$ combined unit.

The haemoglobin $\alpha_2\beta_2$ "molecule" can dissociate into haemoglobin $\alpha\beta$ "dimer" which is rapidly excreted through the kidneys. In discussing pyridoxalation of haemoglobin, Benesch, R. and Benesch, R. E., (*Methods in Enzymology*, **76**, 1981, pp 147-159), reference is made to the possibility of using 2-nor-2-formylpyridoxal-5'-phosphate to prevent such dissociation.

Various patents refer to intramolecular cross-linking of haemoglobin including US-A-4 519 719 which uses a bis-diaspirin ester and also includes a discussion of modification of the oxygen affinity of haemoglobin using pyridoxal-5'-phosphate.

De Venuto and Zegna, *Journal of Surgical Research* **34**, 205-212 (1984), disclosed that polymerising haemoglobin without first pyridoxalating it yielded a product which had increased affinity for oxygen i.e. that pyridoxalating haemoglobin after it had been cross-linked yielded a product that had a higher affinity for oxygen than unmodified haemoglobin. Increased oxygen affinity, reflected in lower $P_{50}$ value (partial pressure of oxygen, usually reported as mm Hg or torr, at which 50% of the haemoglobin is in the oxygenated state) means such a product would not be useful clinically since it would not give up its oxygen readily in body tissues. Thus unmodified stroma-free haemoglobin might show a $P_{50}$ of about 15 mm Hg whereas intermolecular cross-linked haemoglobin may exhibit a $P_{50}$ of only about 9 mm Hg. However De Venuto et al found that haemoglobin subjected to pyridoxalation followed by intermolecular cross-linking may display a $P_{50}$ of about 25 mm Hg.

Further discussion of stroma-free haemoglobin occurs in US-A-4 857 636 which proposes use of dialdehydes to attain conformational and intramolecular stabilisation of haemoglobin by covalent bonding. This is intended to overcome the reported renal excretion of $\alpha\beta$ sub-unit dimers when using pyridoxal-5'-phosphate to modify oxygen affinity. It is suggested that a $P_{50}$ of up to 30 mm Hg is attainable using glutaraldehyde.

Use of dialdehydes to couple deoxy-haemoglobin molecules together or to other physiological proteins under oxygen-free conditions (e.g. nitrogen flushing) was also proposed in US-A-4 336 248. After some three weeks of storage the coupled proteins could be treated with pyridoxal phosphate to obtain a $P_{50}$ of up to 17 mm Hg. This figure, which shows that product to retain a significant oxygen affinity, seems to agree with the findings of De Venuto and Zegna mentioned above who reported the importance of carrying out pyridoxalation before polymerisation to maintain adequate i.e. acceptable $P_{50}$.

Other workers currently active in the field have proposed, as an alternative to polymerisation which gives higher viscosities, stroma-free haemoglobin cross-linked with 2-nor-2-formyl-pyridoxal-5'-phosphate (NFPLP) which is reported to decrease oxygen affinity and block dissociation of the tetramer (*Transfusion*, **27**, 1987, pp 425 - 430, J van der Plas *et al*). The reported $P_{50}$ was in the range of from 26-38 mm Hg for 60-80% coupling mixture Hb/HbNFPLP. Further work reported in *Transfusion*, **28**, 1988, pp 525-530, J van der Plas *et al*, seems to confirm use of that agent provides covalent binding between the 2β sub-unit chains in haemoglobin and suggests a preference for that agent over other polymerisation means yielding a heterogeneous mixture of higher viscosity.

The use of α-hydroxy aldehydes for intermolecular cross-linking of deoxyhaemoglobin is the subject of speculation in a paper presented in *Proc. Natl. Acad. Sci. USA*, **80**, June 1983, pp. 3590-3594, reporting more general work on the reaction of glycolaldehyde with proteins.

It is significant that the prior art methods mainly teach processing of deoxyhaemoglobin (c.f. US-A-4 336 248, US-A-4 529 719 and US-A-4 857 636). It would be a distinct advantage to have available a procedure which was more flexible with respect to the conditions and materials available for its use.

An object of the present invention is to obviate or mitigate the aforesaid difficulties relating to blood substitutes and their use. In particular an object of the present invention is to provide a means of obtaining a stable oxygen transport medium suitable for use as a blood substitute or plasma expander, based on haemoglobin, which overcomes or alleviates some or all of the problems outlined above. A further object of the invention is to obtain a blood replacement or plasma expander which is pasteurisable. It is also an object of this invention to provide a simplified and refined method of obtaining cross-linked, oxygen-affinity modified haemoglobin.

Thus according to this invention there is provided an oxygen transport medium obtainable by treating a haem protein with a primary cross-linking reagent to achieve a significant level of intra- and intermolecular polymerisation and modifying the polymerising protein to attain an acceptable level of oxygen affinity wherein the modification is carried out during polymerisation or as a sequential step.

The haem protein used to form the basis of the oxygen transport medium may be oxyhaemoglobin or deoxyhaemoglobin.

The polymerisation and modification is normally accomplished in accordance with the invention in less than 48 hours and is achievable in about 2 hours.

The preferred primary cross-linking reagent is a monoaldehyde, particularly an α-hydroxy aldehyde such as glycolaldehyde (hydroxyethanal) and the most preferred modifier is an aldehydic multivalent anion such as pyridoxal phosphate, or glyoxalic acid (CHOCOOH).

It has now been found that either carrying out the cross-linking and pyridoxalation reactions together or sequentially in short order yields a modified haemoglobin which is cross-linked and has a decreased oxygen affinity compared to unmodified haemoglobin.

Thus in essence the invention particularly provides a method for making an oxygen carrying plasma expander from haem protein characterised in that said method comprises either (a) the separate sequential steps of firstly cross-linking the haem protein to polymerise same and secondly reducing the affinity for oxygen of the haemoglobin or said method includes (b) one step of cross-linking the haemoglobin and reducing the affinity of the haemoglobin for oxygen during cross-linking.

To amplify this further the invention is characterised by use of a minimum of two reagents, a cross-linker and a modifier, which are brought into contact with the haem protein to be treated such that cross-linking is initiated but not necessarily complete before the modifier is added.

The primary cross-linking reagent will typically achieve well in excess of 70% coupling to form intramolecular cross-linked monomer and oligomers of haemoglobin, i.e. dimers, trimers, tetramers etc.

Since careful reading of some of the prior art references may be necessary to verify the meaning of the term "cross-linking" (i.e. intra- versus intermolecular bonding), for the avoidance of doubt it is hereby stated that in describing this invention the term "cross-linking" includes both polymerisation of haemoglobin molecules to form dimers, tetramers etc and also the bridging of the α and β sub-units which make up a haemoglobin molecule so that these four sub-units are covalently bonded to each other and cannot readily dissociate themselves from each other. Usually the four sub-units in a haemoglobin molecule are bound together by non-covalent bonding which can be disrupted by placing the haemoglobin molecule into 1 M $MgCl_2$ thus allowing the four sub-units to become separated into two dimers.

1 M $MgCl_2$ will not disrupt the covalent bonds formed by cross-linking as disclosed herein, and is used to test how effectively the cross-linking has been carried out. This test essentially comprises placing the haemoglobin, after it has been cross-linked and pyridoxalated, into 1 M $MgCl_2$ and then measuring the molecular weight (MW) by gel filtration of the resultant modified haemoglobin. Ideally this MW for the modified haemoglobin of the present invention will lie in the range of from about 64,000 - 1,000,000.

The molecular weight of the four sub-units of the haemoglobin molecule is about 64,000 and thus the presence of product having a molecular weight of less than 64,000 would indicate that the individual sub-units in the haemoglobin molecule had not been cross-linked, i.e. αβ dimer present (MW 32,000). Such low molecular weight material if present after cross-linking and pyridoxalation would ideally be removed to give a final modified haemoglobin with a MW of between about 64,000 to 1,000,000 since modified haemoglobin having a lower molecular weight will tend to be excreted.

Preferably when cross-linking with a reagent such as glycolaldehyde a suitable range of final concentrations is 0.1-0.4% by weight i.e. 0.1-0.4 g/dl.

The cross-linking reaction of the haem protein, for example haemoglobin, can be carried out in any suitable vessel although preferably it will be carried out in a vessel which will allow the gas content of the reactants to be controlled. Typically the cross-linking, using an $\alpha$ hydroxy aldehyde, is carried out at a pH in the range of from 6 to 9 preferably from pH 7.5 to 8.9.

The temperature of the reaction is preferably kept in the range of from about 4 to 40°C but is most conveniently left at room temperature or thereabouts and most preferably carried out at about 20°C.

The reaction is preferably allowed to proceed for between 2 and 48 hours, suitably for about 7 hours but more conveniently for about 5 hours when using glycolaldehyde.

Typically when using cross-linkers according to the known art the reaction is normally better carried out in an oxygen free or oxygen depleted environment. This can be achieved by, for example, by the use of sodium dithionite (sodium hydrosulphite) or bubbling nitrogen, or another suitable inert gas, through the reaction mixture prior to and/or during the cross-linking reaction. It has been found, surprisingly, that when using glycolaldehyde in accordance with this invention, as the primary cross-linking agent that a low oxygen environment is not absolutely necessary for a fast and complete reaction. Instead it is possible to gradually reduce the oxygen concentration during the cross-linking reaction, instead of prior to the cross-linking reaction which thus allows for a quicker more efficient process.

Further according to this invention it has been found, unexpectedly, that the use of a secondary cross-linking reagent, which is preferably also a modifier of oxygen affinity, in combination with the primary cross-linking reagent referred to above gives improved results. The cross-linking with the primary cross-linker such as, for example glycolaldehyde, can be carried out either before, or simultaneously with modification of the protein by a secondary cross-linking reagent i.e. a combined oxygen affinity regulator and anti-dissociation compound.

Suitable secondary cross-linking reagents or dual-purpose oxygen affinity regulators and cross-linking reagents include for example 2-nor-formyl pyridoxal phosphate, bis (3,5-dibromo-salicyl) fumarate, bis pyridoxal tetraphosphate and 4,4'-diisothiocyanatostilbene-2-2' disulphonate.

This use of a secondary cross-linking reagent, which is also a modifier of oxygen affinity, in combination with a primary cross-linking reagent gives an improved process and a product of that process which has the following beneficial features.

Firstly the chemistry of the two steps are entirely compatible with each other and can be used in sequence or combination. Secondly the oxygen affinity, when haemoglobin is used as the protein, of the treated haemoglobin is in the appropriate physiological range if that haemoglobin were to be used in a physiological replacement fluid. Thirdly the percentage of low molecular weight material is much lower than would be expected if either the primary cross-linking reagent or the secondary cross-linking reagent, which is also a modifier of oxygen affinity, had been used individually.

With regard to modifying the protein, for example haemoglobin, to reduce its oxygen affinity, typically by using a pyridoxalation reaction it has been found that this reaction can either be carried out after the cross-linking reaction has been completed or alternatively it can be carried out essentially simultaneously with the cross-linking reactions. If carried out essentially simultaneously then it is preferably done towards the end of the cross-linking reactions, when the oxygen content is at or near its lowest.

The pyridoxalation reaction, using an aldehydic multivalent anion such as pyridoxal phosphate, has to be carried out in a low oxygen environment. As discussed above this environment is usually achieved by de-gassing, for example, by gas exchange with nitrogen, or treatment with sodium dithionite. Oxygen can be removed from the reaction mixture by addition of a reducing agent such as sodium borohydride or ascorbic acid, towards the end of cross-linking.

In addition the reducing agent converts any iron, in Methaemoglobin, from the $Fe^{3+}$ state which may form during the cross-linking reaction (a form which will not bind oxygen) to the $Fe^{2+}$ state which will then bind oxygen.

Typically the aldehydic multivalent anion is added to the cross-linked protein for example haemoglobin to a final molar ratio of between about 2 to 6 to 1 aldehydic multivalent anion:haemoglobin. This reaction is then left to run, under broadly similar condition of pH, and temperature as those for the cross-linking reaction, for a further suitable period of time, typically 30-60 minutes before the reaction is stopped.

The resultant product can then be purified using known methods and then stored until used. The ability to carry out the cross-linking and pyridoxalation reactions either simultaneously or sequentially allows for a quick reaction which can be carried out in one vessel thus reducing the time and cost for producing the final product compared with known reactions. Furthermore the polymerisation is easily controlled to give the desired molecular weight of the final product.

The oxygen affinity of the oxygen transport medium obtainable according to the present invention would be less than that for unmodified stroma-free haemoglobin which has typically a $P_{50}$ of 5-15 mm Hg. Preferably the $P_{50}$ of the oxygen transport medium of the present invention will lie in the range of 20 to 35 mm Hg and more preferably from 25 to 30 mm Hg rendering it eminently suitable as a plasma expander.

It will be appreciated by those in this art that whilst the examples used hereinbelow to illustrate invention refer to haemoglobin, the present invention can apply to other suitable proteins and peptides having similar functions and properties.

It will be further understood that whilst the present examples disclose substantially homogeneous oxygen transport medium i.e. haemoglobin cross-linked to haemoglobin, this invention is applicable to heterogeneous material wherein the haemoglobin would be cross-linked to another suitable high MW molecule such as another compatible protein for example.

The invention will now be further described by way of the following examples which are illustrative and not exhaustive.

## 1. Preparation of Novel Stable Oxygen Carrying Plasma Expander

Eight to ten units of outdated human red cells were washed four times with isotonic saline (IBM 2991 cell washer). Six volumes of packed cells were then lysed by addition of four volumes of sterile, de-ionised water. Stroma-free haemoglobin (Hb) was then prepared by chloroform extraction of the red cell lysate.

### Polymerisation (Cross-linking) and Pyridoxalation

All operations, except where indicated, were carried out at room temperature and under sterile conditions. For example, the Hb solutions were concentrated to 14 g/dl using a hollow fibre dialysis cartridge (10 kD pore size; Cobe Centrisystem 400) with an applied back pressure of 8-9 psi and pH was adjusted to pH 8.5 using 1 M $Na_2CO_3$.

Polymerisation was carried out using glycolaldehyde (Sigma). A 10% aqueous stock solution of glycolaldehyde was prepared and 3.13 ml was added per 100 ml of 14 g/dl Hb solution to give a final concentration of 0.3%.

Polymerisation was monitored by colloid oncotic pressure (COP) or fast protein liquid chromatography (FPLC) gel filtration (Superose 12 column: Pharmacia). After 5-6 hours the oxygen content was reduced by gas exchange with a hollow fibre cartridge, the pH was adjusted to pH 7.2 using 1 M $NaHCO_3$, and pyridoxal phosphate (PLP) as a 4.08% solution of PLP prepared in 1 M Tris buffer pH 7.2, was taken and 7.5 ml added per 100 ml of 14 g/dl solution to give a molar ratio 6:1 PLP/Hb. Both reactions were allowed to proceed for a further 30 minutes.

In order to stabilise Hb-PLP covalent bonds and terminate the polymerisation process, sodium borohydride ($NaBH_4$; Sigma) was then added. A 3.12% solution was prepared in $10^{-3}$ M NaOH; 5 ml of this solution were added per 100 ml of 14 g/dl Hb solution to give a molar ratio of 20:1 $NaBH_4$:Hb and left overnight at 4°C. Air was then readmitted to the reaction vessel and the Hb solution dialysed extensively against deionised water using a kidney dialysis cartridge (pore size 10 kD) to remove excess reagents and red cell-derived low molecular weight contaminants. Simultaneous concentration was achieved by applying a back pressure of 8-9 psi and the solution finally dialysed against Krebs' solution pH 7.4, before 0.22 μm filtration (Gelman Sciences) into sterile transfer packs and storage at -40°C.

The extensive dialysis of this procedure has the added benefit of reducing the levels of adenine nucleotides, present in the starting material, to a concentration of less than about 0.3 mg/dl, which means that the final product has little if any vasoactive activity which is thought to be at least partially due to the adenine nucleotides.

The MW range of the cross-linked final product when placed in 1M $MgCl_2$ was between about 64,000 D and 1,000,000 D with a mean value of 300,000 D as determined by analytical gel filtration.

## 2. Primary and Secondary Cross-linking.

A modified protein suitable for use as an oxygen carrier in a physiologic replacement fluid can be prepared from haemoglobin using the following procedure.

Haemoglobin is polymerised or cross-linked with a primary cross-linker such as glycolaldehyde at 0.3%, at a pH of 8.5 for three and half hours at 20°C. During or even after this reaction the reaction solution has its pH adjusted to 7.2 and is thoroughly de-oxygenated prior to reaction with a secondary cross-linker which also modifies oxygen affinity. A suitable secondary cross-linker is 2-nor-formyl pyridoxal phosphate in which case it is added to the reaction mixture at a molar ratio of between about 1:1 to 4:1 2-nor-formyl pyridoxal phosphate to haemoglobin.

The low molecular weight excretable dimer, approximately MW 34 kD, is by virtue of this invention dropped from a typical previous value of about 30% down to about 14-19% of the resultant product and the $P_{50}$ of the resultant product is stepped up from circa 12-14 mm Hg to the much more favourable range of about 30-35 mm Hg.

The molecular weight range distribution following this treatment as determined by analytical gel filtration is such that 20-30% of the resultant product has a molecular weight of about 68 kD and about 60% of the product has a MW between 130-500 kD. The percentage of low molecular weight product resulting from this particular reaction is approximately half that of the low molecular weight product which would result from a reaction which involved a primary cross-linking reagent such as glycolaldehyde alone or after reaction involving either glycolaldehyde or pyridoxal phosphate alone. The $P_{50}$ would be in the order of 12-14 mm Hg after glycolaldehyde cross-linking and 22-24 mm Hg after reaction with pyridoxal phosphate.

A significant feature of this invention is that the oxygen transport medium obtainable by the described methods is pasteurisable according to known procedures.

As a comparison with the prior art methods an attempt was made to apply the protocol of this invention to haemoglobin but substituting glutaraldehyde for glycolaldehyde. The conditions were as described above i.e. the glutaraldehyde concentration in haemoglobin was 0.3%, pH was 8.5 and the temperature was about 21°C. It was observed that within 5 minutes of adding glutaraldehyde the haemoglobin had completely solidified within the reaction vessel. Therefore it was completely pointless to consider modification of oxygen affinity so that no pyridoxalation step was carried out.

Whilst the foregoing describes the methods and preparation of a novel oxygen carrier based on haemoglobin it will be apparent to those skilled in this art that these methods are applicable to other proteins and peptides particularly those proteins and peptides which have an affinity for gases.

## Claims

1. A stable oxygen transport medium comprising haem proteins wherein the medium is obtainable by treating a haem protein with a primary cross-linking reagent to achieve a significant level of protein coupling and form polymers thereof, and modifying the polymerising protein to attain an acceptable level of oxygen affinity wherein the modification is carried out during polymerisation or as a sequential step.

2. A medium according to claim 1 wherein the haem proteins comprise oxyhaemoglobin or deoxyhaemoglobin.

3. A medium according to claim 1 or claim 2 wherein the polymerisation and modification is substantially complete in less than 48 hours.

4. A medium according to any one of claims 1 to 3 wherein the primary cross-linking reagent is an $\alpha$-hydroxy aldehyde.

5. A medium according to claim 4 wherein the primary cross-linking reagent is glycolaldehyde.

6. A medium according to any one of claims 1 to 5 wherein the modifier is an aldehydic multivalent anion.

7. A medium according to any one of claims 1 to 5 wherein the modifier is pyridoxal phosphate or glyoxalic acid.

8. A medium according to any one of claims 1 to 7 wherein a secondary cross-linking reagent which is also a modifier of oxygen affinity, is used in combination with the primary cross-linking reagent.

9. A medium according to claim 8 wherein the secondary cross-linking reagent is selected from 2-nor-formyl pyridoxal phosphate, bis (3,5-dibromo-salicyl) fumarate, bis pyridoxal tetraphosphate and 4,4'-diisothiocyanatostilbene-2-2' disulphonate.

10. A method according to any one of claims 1 to 9 wherein the molecular weight of the cross-linked haem protein is in the range of from 64,000 - 1,000,000 D.

11. A method for making an oxygen carrying plasma expander from haem proteins comprising cross-linking same and modifying the oxygen affinity thereof *characterised* by
    (a) the separate sequential steps of firstly cross-linking the haem protein to polymerise same and secondly reducing the affinity for oxygen of the haem protein by the addition of a modifier; or
    (b) one step of cross-linking the haemoglobin and reducing the affinity of the haem protein for oxygen by the presence of a modifier during cross-linking.

12. A method according to claim 11 wherein a secondary cross-linking reagent which is also a modifier of oxygen affinity, is used in combination with the primary cross-linking reagent.

13. A method according to claim 12 wherein the secondary cross-linking reagent is added before, simultaneously or after treatment of the haem protein by the primary cross-linking reagent.

14. A method according to any one of claims 11 to 13 wherein the secondary cross-linking reagent is selected from 2-nor-formyl pyridoxal phosphate, bis (3,5-dibromo-salicyl) fumarate, bis pyridoxal tetraphosphate and 4,4'-diisothiocyanatostilbene-2-2' disulphonate.

15. A method according to claim 14 wherein the secondary cross-linking reagent is 2-nor-formyl pyridoxal phosphate which is used in a molar ratio of 2-nor-formyl pyridoxal phosphate to haemoglobin of from 1:1 to 4:1.

16. A method according to any one of claims 11 to 15 wherein the primary cross-linking reagent is an a-hydroxy aldehyde.

17. A method according to claim 16 wherein the primary cross-linking reagent is glycolaldehyde.

18. A method according to any one of claims 11 to 17 wherein the modifier is pyridoxal phosphate or glyoxalic acid.

19. A method according to any one of claims 11 to 18 wherein oxygen is removed during the course of cross-linking the haem protein to reduce the amount of oxygen present to a level enabling the addition of a modifier for reducing the affinity for oxygen of the haem protein.

20. A method according to any one of claims 11 to 19 wherein the cross-linked and modified haem protein is pasteurised.